Europäisches Patentamt

European Patent Office

Office européen des brevets

(19)

(11) Veröffentlichungsnummer: **0 000 778**
**B1**

(12) **EUROPÄISCHE PATENTSCHRIFT**

(21) Anmeldenummer: **78100591.3**

(22) Anmeldetag: **04.08.78**

(51) Int. Cl.³: **C 07 C 87/50,**
**C 07 C 85/24**

(54) Verfahren zur Herstellung von Polyaminen der Diphenylmethanreihe

(30) Priorität: **16.08.77 DE 2736862**

(43) Veröffentlichungstag der Anmeldung:
**21.02.79 Patentblatt 79/04**

(45) Bekanntmachung des Hinweises auf die Patenterteilung:
**23.07.80 Patentblatt 80/15**

(84) Benannte Vertragsstaaten:
**BE DE FR GB NL**

(56) Entgegenhaltungen:
**DD - A - 109 615**
**DE - A - 2 037 550**
**DE - B - 1 179 945**
**DE - C - 1 210 872**
**FR - A - 2 370 032**
**US - A - 3 367 969**
**US - A - 3 971 829**

(73) Patentinhaber: **Bayer AG**
**Zentralbereich Patente, Marken und Lizenzen**
**D - 5090 Leverkusen 1, Bayerwerk (DE)**

(72) Erfinders: **Neumann, Rainer, Dr.**
**Bodelschwinghstrasse 16**
**D - 4150 Krefeld (DE)**
**Schwarz, Hans-Helmut, Dr.**
**Ratherstraat 90**
**D - 4150 Krefeld 1 (DE)**
**Heuser, Jürgen, Dr.**
**Doeperhofstrasse 35**
**D - 4150 Krefeld (DE)**

Courier Press, Leamington Spa, England.

Verfahren zur Herstellung von Polyaminen der Diphenylmethanreihe

Polyamine der Diphenylmethanreihe entstehen als Kondensationsprodukte aus Anilin und Formaldehyd an sauren Katalysatoren. Zunächst reagieren Anilin und Formaldehyd auch ohne Katalysatoren zu N-Alkylverbindungen, die als Vorkondensat bezeichnet werden. Dieses Vorkondensat lagert in Gegenwart von sauren Katalysatoren in Polyamine der Diphenylmethanreihe um. Dabei entsteht eine Mischung aus 2,2'-, 2,4'-, 4,4'-Diaminodiphenylmethanen und höheren Kondensationsprodukten (Dreikern- bis Sechskernverbindungen). Durch Umsetzen dieser Verbindungen mit Phosgen erhält man die entsprechenden Polyisocyanate der Diphenylmethanreihe, die wertvolle Ausgangsmaterialien bei der Herstellung von Polyurethankunststoffen insbesondere Polyurethanschaumstoffen darstellen. Für viele Anwendungsgebiete dieser Polyisocyanatgemische ist es von großem Interesse, daß der in ihnen vorliegende Anteil an orthoständigen Isocyanatgruppen, insbesondere der Anteil an 2,2'- und 2,4'-Diisocyanatodiphenylmethan, möglichst neidrig ist. Dementsprechend strebt man bereits bei der Anilin/Formaldehyd-Kondensation Polyamingemische der Diphenylmethanreihe an, die diesen Voraussetzungen bezüglich der Isomerenverteilung entsprechen. Bei Verwendung von Salzsäure als Katalysator ist es zwar möglich, den Gehalt an o-Isomeren im Kondensationsgemisch niedrig zu halten, jedoch ist die Verwendung von Salzsäure (bzw. von Anilinhydrochlorid) mit den Nachteilen der Korrosivität, sowie der Notwendigkeit einer Neutralisation bzw. aufwendigen Abtrennung des Katalysators vom Reaktionsprodukt verbunden. Diese Nachteile können zwar im Prinzip durch den Einsatz heterogener saurer Katalysatoren, wie z.B. Ionenaustauscher vermieden werden, doch ist es bei Verwendung dieser Katalysatoren bisher nicht möglich gewessen, Polyamine der Diphenylmethanreihe herzustellen, die beispielsweise ein Gehalt von weniger als 12 % an 2,4'-Diaminodiphenylmethan und einen Gehalt von weniger als 2 % an 2,2'-Diaminodiphenylmethan aufweisen. So wird beispielsweise gemäß DDR-Wirtschaftspatent 109 615 an organischen sauren Ionenaustauschern ein Polyamingemisch der Diphenylmethanreihe erhalten, welches 24—26 Gew.-% 2,4'- und ca. 2,8 Gew.-% 2,2'-Diaminodiphenylmethan enthält.

Auch nach der DT-OS 2 037 550 ist die Herstellung von o-Isomeren-armen Verfahrensprodukten, wie den Ausführungsbeispielen dieser OS zu entnehmen, nicht möglich.

Folgt man dem Hinweis der genannten OS, die Isomerenverteilung über den Wassergehalt des Kondensationsgemisches zu steuern, so legen es die Versuche zum Erreichen eines niedrigen 2,4'-Diaminodiphenylmethananteils nahe, weitestgehend wasserfrei zu arbeiten. Insbesondere Versuche (Beispiele 1 und 2), die durch Entfernung des Kondensationswassers einen niedrigeren Gesamtwassergehalt als die anderen Versuche haben, führen zu relativ niedrigen Anteilen an 2,4'-Isomeren (17,9—25 Gew.-%, vergleichbar mit dem DDR-Wirtschaftspatent 109 615). Durch vergleich mit den restlichen Beispielen (3—5) der OS, bei denen das Kondensationswasser nicht entfernt wurde und 2,4'-Isomerenanteile des Diaminodiphenylmethans von bis zu 85 Gew.-% erzielt wurden, wird erkennbar, daß zur Herstellung eines Produktes mit hohem 4,4'- und niedrigem 2,2'- und 2,4'-Diaminodiphenylmethananteil möglichst wasserfrei gearbeitet werden muß.

Gemäß den Ausführungsbeispielen der DT-AS 1 179 945 werden zwar hohe Ausbeuten an 4,4'-Isomeren erhalten jedoch wird in diesen Beispielen keine Angabe über den Gehalt an 2,2'- und 2,4'-Isomeren gemacht, so daß vermutet werden muß, daß die angegebenen Ausbeuten an 4,4'-Isomeren tatsächlich die Gesamtausbeuten an 2,2'-, 2,4'- und 4,4'-Isomeren darstellen. Diese Vermutung wird im übrigen auch durch das nachstehende Vergleichsbeispiel 5 gestützt, mit welchem nachgewiesen wird, daß bei destillativer Entfernung des Wassers während der Kondensationreaktion ein Polyamingemisch mit einem hohen Anteil an 2,2'- und 2,4'-Isomeren erhalten wird.

Überraschenderweise wurde nunmehr gefunden, daß bei Verwendung ganz bestimmter, nachstehend näher beschriebener, saurer Ionenaustauscher dann Polyamingemische der Diphenylmethanreihe mit einem niedrigen Gehalt an 2,2'- und 2,4'-Isomeren erhalten werden können, wenn in Überwindung des durch die Lehre der DT-OS 2 037 550 begründeten Vorurteils die speziellen Katalysatoren in Wasser gesättigtem Zustand zur Anwendung gelangen.

Gegenstand der vorliegenden Erfindung ist ein Verfahren zur Herstellung von Polyaminen der Diphenylmethanreihe mit einem hohen Gehalt an 4,4'-Isomeren und einem niedrigen Gehalt an 2,2'- und 2,4'-Isomeren durch Umsetzung von primären oder sekundären aromatischen Aminen mit Formaldehyd in Gegenwart von in heterogener Phase vorliegenden, festen, sauren Katalysatoren, gegebenenfalls über die Zwischenstufe von in Abwesenheit von sauren Katalysatoren hergestellten N - substituierten Vorkondensaten, dadurch gekennzeichnet, daß man

a) als Katalysator wassergesättigte, Sulfonsäuregruppen - aufweisende, gelförmige Ionenaustauscher auf Basis von Styrol - divinylbenzol -copolymerisaten, welche mit 2 Gew.-%, bezogen auf Copolymerisat an Divinylbenzol, vernetzt sind, oder wassergesättigte, Sulfonsäuregruppen aufweisende, makroporöse Ionenaustauscher auf Basis von Styrol - divinylbenzol - copolymerisaten, die mit 18 Gew.-%, bezogen auf Copolymerisat, an Divinylbenzol vernetzt sind, verwendet und

b) man die katalytische Umsetzung im Temperaturbereich von 50—150°C gegebenenfalls unter Druck ohne nennenswertes Abdestillieren des Kondensationswassers und des in das System eingebrachten Wassers durchführt. Unter "Polyaminen der Diphenylmethanreihe" sind im Rahmen der vorliegenden Erfindung sowohl Polyamingemische zu verstehen, wie sie bei der Kondensation von Anilin selbst mit Formaldehyd in Gegenwart von Säurekatalysatoren entstehen, als auch solche Polyamingemische wie sie durch Säurekatalysierte Kondensation von am Stickstoff oder am Kern substituierten Anilinen mit Formaldehyd erhalten werden. Die bevorzugten erfindungsgemäßen Polyamine der Diphenylmethanreihe sind jedoch die klassischen Anilin/Formaldehyd - Kondensate.

Diesen Ausführungen entsprechend können beim erfindungsgemäßen Verfahren als Ausgangsmaterialien beliebige, als substituierte Aniline aufzufassende primäre oder sekundäre Arylamine eingesetzt werden. Voraussetzung für die Eignung der Amine als Ausgangsmaterial beim erfindungsgemäßen Verfahren ist lediglich, daß die gegebenenfalls vorliegenden Substituenten unter den Reaktionsbedingungen des erfindungsgemäßen Verfahrens inert sind, und daß in den Aminen eine zu einer aromatisch gebundenen Aminogruppe unsubstituierte para-Stellung vorliegt. Als Ausgangsmaterialien geeignete Arylamine sind demzufolge N-Methylanilin, N-Äthylanilin, o-Toluidin, o-Chloranilin, m-Chloranilin, o-Anisidin, 2,3-Xylidin, 3,5-Xylidin, o-Cyclohexylanilin, o-Benzylanilin, $\alpha$-Naphthylanilin, Methylmercaptoanilin oder das im Rahmen der vorliegenden Erfindung besonders bevorzugte Anilin.

Der beim erfindungsgemäßen Verfahren als Reaktionspartner für das Arylamin einzusetzende Formaldehyd wird vorzugsweise als wäßrige Formalinlösung eingesetzt, obwohl prinzipiell auch gasförmiger Formaldehyd oder unter den Reaktionsbedingungen Formaldehyd abspaltende Stoffe wie z.B. Paraformaldehyd oder Trioxymethylen geeignet sind.

Bei den erfindungswesentlichen Katalysatoren handelt es sich um saure, Sulfonsäuregruppen aufweisende Ionenaustauscher auf Basis von Styrol - divinylbenzol - copolymerisaten, wie sie durch Mischpolymerisation von Styrol mit Divinylbenzol und anschließender Sulfonierung mit Schwefelsäure bzw. Oleum gemäß bzw. in Analogie zu den in US-PS 2 366 007; J. Appl. Chem. 1, 124 (1951); DT-PS 908 247; BE-PS 500 370; DT-AS 1 168 081, erhalten werden, welche im allgemeinen pro aromatischem Ring 0,8-1,2 vorzugsweise eine Sulfonsäuregruppe aufweisen und welche entweder

a) gelförmige Ionenaustauscher, die mit 2 Gew.-% an Divinylbenzol, bezogen auf Styrol-divinylbenzol-copolymerisat, vernetzt sind, oder welche

b) makroporöse Ionenaustauscher, die mit 18 Gew.-% an Divinylbenzol, bezogen auf Styrol - divinylbenzol-copolymerisat, vernetzt sind,

darstellen. Außer den genannten Kriterien is für die Eignung der Katalysatoren wesentlich, daß sie in wassergesättigtem Zustand zur Anwendung gelangen. Die Menge der beim erfindungsgemäßen Verfahren einzusetzenden Katalysatoren wird im allgemeinen so gewählt, daß bei diskontinuierlicher Arbeitsweise im Reaktionsgemisch pro 100 Gew.-Teilen an Ausgangs-arylamin 1 bis 50 vorzugsweise 1 bis 25 Gew.-Teile des Katalysators (bezogen) auf Trockensubstanz) vorliegen. Bei kontinuierlicher Arbeitsweise beispielsweise unter Verwendung eines Kontaktbetts werden die Katalysatoren im allgemeinen in solchen Mengen eingesetzt, daß pro 1 Kilogramm/Stunde an zum Einsatz gelangenden Arylamins 3 bis 30 vorzugsweise 10 bis 20 kg des Kontakts zur Verfügung stehen. Die Verweilzeit des zum Einsatz gelangenden Arylamins am Katalysator beträgt zwischen 3 und 30 Stunden. In jedem Fall empfiehlt sich jedoch, die optimale Menge des jeweils zum Einsatz gelangenden Katalysators durch einen entsprechenden Vorversuch zu ermitteln.

Die Katalysatoren können in verschiedener äußerer Beschaffenheit zur Anwendung gelangen, z.B. in stückiger Form, in Form von handelsüblichem Perlgranulat oder auch in fein gemahlenem Zustand. Eine Aktivierung des Katalysators kann gegebenenfalls in an sich bekannter Weise durch Säurebehandlung erreicht werden.

Beim erfindungsgemäßen Verfahren wird das Molverhältnis Arylamin : Formaldehyd im allgemeinen im Bereich von 3 : 1 bis 15 : 1, vorzugsweise 5 : 1 bis 10 : 1, gehalten. Im allgemeinen wird das erfindungsgemäße Verfahren so durchgeführt, daß die wäßrige Formaldehydlösung bzw. der Formaldehyd abgebende Stoff zum aromatischen Amin gegeben wird, wobei auf gute Durchmischung und Abfuhr der Reaktionswärme geachtet werden sollte. Das aromatische Amin wird zweckmäßigerweise gleich in den für die weitere Reaktionsführung gewünschten Überschuß angewendet und dient gleichzeitig als Lösungsmittel für das entstehende N-substituierte Vorkondensat, wobei u.U. durch zusätzliche Zugabe von Wasser das Reaktionsgemisch auf jeden Fall wassergesättigt vorliegen muß.

Das wassergesättigte Reaktionsgemisch wird nun unter Verwendung der o.g. Katalysatoren bei erhöhter Temperatur weiter umgesetzt, wobei sich aus dem Vorkondensat und einem Teil des im Überschuß vorliegenden Amins das erfindungsgemäße Verfahrensprodukt bildet. Selbstverständlich ist es auch möglich, den Katalysator mit den Ausgangsmaterialien schon von Anfang an zusammenzubringen, so daß bereits die Bildung der N-substituierten Vorkondensate in Gegenwart der Katalysatoren abläuft.

Das erfindungsgemäße Verfahren wird insbesondere während der erfindungsgemäße katalysierten Umlagerung der N-substituierten Zwischenprodukte in die erfindungsgemäßen Verfahrensprodukte bei erhöhten Temperaturen im allgemeinen im Temperaturbereich von 50 bis 150°C durchgeführt. Es ist jedoch darauf zu achten, daß Druck- und Temperaturverhältnisse so gewählt werden, daß ein nennenswertes Abdestillieren des in das Reaktionsgemisch eingebrachten Wassers und des Kondensationswassers unterbleibt, so daß stets sichergestellt bleibt, daß der Katalysator im wassergesättigten Zustand vorliegt. So ist es wesentlich, daß beim Arbeiten bei über 100°C liegenden Temperaturen ein ein Abdestillieren des Wassers verhindernder Druck aufrechterhalten wird. Es kann oft von Vorteil sein, das erfindungsgemäße Verfahren unter zweistufiger Temperaturführung durchzuführen, wobei die Bildung der N - substituierten Vorkondensate im allgemeinen im Temperaturbereich von 20 bis 50°C und die katalytische Umlagerung dieser Vorkondensate in die erfindungsgemäßen Verfahrensprodukte im Temperaturbereich von 50 bis 120°C erfolgt.

Das erfindungsgemäße Verfahren kann kontinuierlich in einem Kontaktbett, einem Rührkessel oder einer Rührkesselkaskade oder diskontinuierlich im Rührkessel ausgeführt werden. Da im Kontaktbett die Abtrennung und Rückführung des Katalysators nicht erforderlich ist, empfiehlt sich diese Art der Durchführung.

Zur besseren Reaktionsführung, insbesondere zur Abführung der Reaktionswärme, kann es auch günstig sein, die Reaktion in mehreren hintereinandergeschalteten Stufen ablaufen zu lassen, wobei nach den einzelnen Stufen die jeweils gebildete Reaktionswärme abgefürt werden kann.

Die Aufarbeitung des Reaktionsgemisches erfolgt vorzugsweise destillativ, obwohl prinzipiell jedes andere geeignete Trennverfahren Anwendung finden kann. Das niedriger siedende überschüssige Amin wird über Kopf abdestilliert und in den Prozeß zurückgeführt, während das als Destillationsrückstand vorliegende Polyamingemisch unmittelbar, z.B. durch Phosgenierung, in bekannter Weise zu einem Polyisocyanatgemisch weiter verarbeitet werden kann.

### Beispiel 1

465 Gew.-Tle. Anilin und 43 Gew.-Tle. einer 35 %igen wäßrigen Formaldehydlösung wurden mit 20 Gew.-% (bezogen auf trockenen Zustand) wasserfeuchtem, gelförmigen mit 2 Gew.-% an Divinyl - benzol bezogen auf Divinylbenzol - styrol - copolymerisat vernetztem Ionenaustauscher bestehend aus einem sulfoniertem Styrol - divinylbenzol - copolymerisat, welches pro aromatischen Ring eine Sulfonsäuregruppe enthält in einem mit Rührer, Thermometer und Rückflußkühler versehenen Dreihalskolben bei 95°C zur Reaktion gebracht. Nach 20 h wurde die Reaktionslösung abgetrennt und der Ionenaustauscher mit neuer Ausgangslösung erneut zur Reaktion gebracht. Die Reaktionslösung war dabei jeweils wassergesättigt, was sich durch Trübung der Reaktionslösung bzw. Ausbilden einer separaten Wasserphase erkennen ließ. Nach der 40. Wiederholung des Versuches wurde die Reaktionslösung nach dest. Abtrennung des Anilins hochdruckflüssigkeitschromatographisch untersucht.

Das Polyamingemisch enteilt 0,6 Gew.-% 2,2'-, 10,8 Gew.-% 2,4'- und 75,3 Gew.-% an 4,4'-Diaminodiphenylmethan, der Rest bestand aus 3- bzw. 4-Kernverbindungen.

Die Versuche wurden bis zu einer Gesamtdauer von 3000 h durchgeführt, ohne das sich Umsatz oder Selektivität änderten.

### Beispiel 2

Beispiel 1 wird wiederholt, mit dem einzigen Unterschied, daß eine entsprechende Menge eines wasserfeuchten, makroporösen Ionenaustauschers, der mit 18 Gew.-% Divinylbenzol bezogen auf Divinylbenzol - styrol - copolymerisat vernetzt ist, eingesetzt wurde.

Das erhaltene Polyamingemisch setzte sich aus 0,6 Gew.-% 2,2'-, 8,0 Gew.-% 2,4'- und 73,2 Gew.-% an 4,4'-Diaminodiphenylmethan und höherkernigen Verbindungen zusammen, Auch hier wurden die Versuche wie in Beispiel 1 3000 h durchgeführt, ohne daß sich Umsatz oder Selektivität änderten.

### Beispiel 3 (Vergleich)

Beispiel 1 wird wiederholt, mit dem einzigen Unterschied, daß ein gelförmiger, wasserfeuchter mit 4 Gew.-% Divinylbenzol bezogen auf Divinylbenzol - styrol - copolymerisat vernetzter Ionenaustauscher eingesetzt wurde.

Das erhaltene Polyamingemisch setzte sich aus 1,1 Gew.-% 2,2'- 14,6 Gew.-% 2,4'- und 57,2 Gew.-% an 4,4'-Diaminodiphenylmethan zusammen. Nahezu 20 Gew.-% bestanden aus 3- und höherkernigen Verbindungen.

### Beispiel 4 (Vergleich)

Beispiel 1 wird wiederholt, mit dem einzigen Unterschied, daß ein gelförmiger, wasserfeuchter, mit 8 Gew.-% Divinylbenzol bezogen auf Divinylbenzol - styrol - copolymerisat vernetzer Ionenaustauscher eingesetzt wurde.

Bei diesem Ionenaustauscher wurde der Umsatz von Versuch zu Versuch geringer und nach 20 Versuchen (400 h Gesamtreaktionszeit) wurde nur noch ein unter 50 % liegender Umsatz erzielt. Nach weiteren Versuchen ging der Umsatz bis auf Null zurück, der Ionenaustauscher war auch nach den üblichen Methoden nicht mehr zu regenerieren.

Beispiel 5 (vergleich)

465 Gew.-Tle. Anilin und 43 Gew.-Tle. einer 35 %igen wäßrigen Formaldehydlösung wurden zunächst ohne Katalysator in der in Beispiel 1 beschriebenen Apparatur zur Reaktion gebracht. Das Wasser der wäßrigen Formaldehydlösung und das gebildete Kondensationswasser wurden destillativ bis auf einen unter 0,07 Gew.-% liegenden Wert entfernt. Sodann wurden 20 Gew.-% des in Beispiel 1 erwähnten trockenen Ionenaustauschers hinzugegeben und die Lösung 20 h bei 100°C zur Reaktion gebracht. Nach Beendigung des Versuches wurde die Reaktionslösung abgetrennt und mit neuer trockner Ausgangslösung umgesetzt. Nach jeweils einigen Versuchen wurden auch geringe Mengen des möglicherweise mit eingeschleusten Wassers abdestilliert, um somit immer einen wasserfreien Reaktionsablauf zu haben.

Das gebildete Polyamingemisch enthielt 3,4 Gew.-% 2,2'-, 16,5 Gew.-% 2,4'- und 54 Gew.-% 4,4'-Diaminodiphenylmethan. Der Rest bestand aus höherkernigen Verbindungen.

Die Versuche wurden bis zu einer Gesamtdauer von 3000 h durchgeführt, ohne daß durch Änderung von Reaktionsparametern, wie z.B. Anilin/Formaldehyd-Molverhältnis, Temperatur oder Verweilzeit ein den Beispielen 1 und 2 entsprechendes Produkt erhalten wurde.

## Patentanspruch

Verfahren zur Herstellung von Polyaminen der Diphenylmethanreihe mit einem hohen Gehalt an 4,4'-Isomeren und einem niedrigen Gehalt an 2,2'- und 2,4'-Isomeren durch Umsetzung von primären oder sekundären aromatischen Aminen mit Formaldehyd in Gegenwart von in heterogener Phase vorliegenden, Sulfonsäuregruppen aufweisenden Ionenaustauschern auf Basis von Styrol - Divinylbenzol - Copolymeren als feste Katalysatoren, gegebenenfalls über die Zwischenstufe von in Abwesenheit von sauren Katalysatoren hergestellten N-substituierten Vorkondensaten, dadurch gekennzeichnet, daß man
a) als Katalysator wassergesättigte, Sulfonsäuregruppen aufweisende, gelförmige Ionenaustauscher auf Basis von Styrol - Divinylbenzol - Copolymerisaten, welche mit 2 Gew.-%, bezogen auf Copolymerisat, an Divinylbenzol vernetzt sind, oder wassergesättigte, Sulfonsäuregruppen aufweisende, makroporöse Ionenaustauscher auf Basis von Stryol - Divinylbenzol - Copolymerisaten, die mit 18 Gew.-%, bezogen auf Copolymerisat, an Divinylbenzol vernetzt sind, verwendet und
b) man die katalytische Umsetzung im Temperaturbereich von 50—150°C gegebenenfalls unter Druck ohne nennenswertes Abdestillieren des Kondensationswassers und des in das System eingebrachten Wassers durchführt.

## Revendication

Procédé de production de polyamines de la série du diphénylméthane à teneur élevée en isomère 4,4' et à faible teneur en isomères 2,2' et 2,4' par réaction d'amines aromatiques primaires ou secondaires avec le formaldéhyde en présence d'échangeurs ioniques en phase hétérogène, portant des groupes acide sulfonique, à base de copolymères styrène-divinylbenzène, comme catalyseurs solides, éventuellement avec passage par le stade intermédiaire de précondensats substitués sur l'atome d'azote produits en l'absence de catalyseurs acides, caractérisé en ce qu'il consiste:
a) à utiliser comme catalyseur des échangeurs ioniques saturés d'eau sous forme de gel, présentant des groupes acide sulfonique, à base de copolymères de styrène et de divinylbenzène qui sont réticulés avec 2 % en poids de divinylbenzène, par rapport au produit de copolymérisation, ou des échangeurs ioniques saturés d'eau, à pores macroscopiques, portant des groupes acide sulfonique, à base de copolymères de styrène et de divinylbenzène qui sont réticulés avec 18 % en poids de divinylbenzène par rapport au produit de copolymérisation et
b) à conduire la réaction catalytique dans la plage de températures de 50 à 150°C, le cas échéant sous pression, sans élimination notable par distillation de l'eau de condensation et de l'eau introduite dans le système.

## Claim

A process for the production of polyamines of the diphenyl methane series having a high content of 4,4'-isomers and a low content of 2,2'- and 2,4'-isomers by reacting primary or secondary aromatic amines with formaldehyde in the presence of ion exchangers as solid catalysts, which ion exchangers contain sulphonic acid groups, are based on styrene-divinyl benzene copolymers and are present in heterogeneous phase, optionally by way of the intermediate stage of N-substituted precondensates produced in the absence of acid catalysts, characterised in that
a) the catalysts used are water-saturated gelform ion exchangers containing sulphonic acid groups based on styrene-divinyl benzene copolymers, which are cross-linked with 2%, by weight, based on copolymer, of divinyl benzene, or water-saturated macroporous ion exchangers containing sulphonic acid groups based on styrene-divinyl

benzene copolymers which are cross-linked with 18%, by weight, based on copolymer, of divinyl benzene; and

b) the catalytic reaction is carried out within a temperature range from 50—150°C optionally under pressure and without any significant distillation of the condensation water and the water introduced into the system.